# EUROPEAN PATENT APPLICATION

(11) **EP 2 514 354 A1**
(43) Date of publication of application: **24.10.2012**
(21) Application number: 12163864.7
(22) Date of filing: 12.04.2012
(51) Int. Cl.: A61B 1/00, A61B 1/07

(54) **Trocar with integrated light and/or scope optical fibers**

(30) Priority: 18.04.2011 US 201161476408 P; 09.03.2012 US 201213415870
(71) Applicant: Tyco Healthcare Group LP, Mansfield, MA 02048 (US)
(72) Inventor: Menn, Dmitri, Marblehead, MA Massachusetts 01945 (US)
(74) Representative: Soames, Candida Jane

(57) **Abstract**

A trocar assembly having integrated optical scope and light fibers is provided for illuminating and visualizing an operative site. The trocar assembly includes an obturator and an illuminated optical access port. An optical fiber extends through the walls of the access port from a distal end of the access port to a receptacle or connector located on a housing of the access port. The access port additionally includes one or more illuminating light fibers which also extend through the side walls of the access port from the distal end of the access port to the receptacle or connector on the housing.

## Description

### CROSS REFERENCE TO RELATED APPLICATION

The present application claims the benefit of and priority to U. S. Provisional Application Serial No.61/476,408, filed on April 18, 2011, the entire contents of which are incorporated herein by reference.

### BACKGROUND

### 1. Technical field

The present disclosure relates to a trocar assembly having integrated light and optical scope fibers. More particularly, the present disclosure relates to a trocar or access port incorporating illuminating light fibers and one or more optical viewing scope fibers integrated into the side walls of the access port.

### 2. Background Of Related Art

During minimally invasive surgical procedures access ports or trocar assemblies are provided to penetrate an abdominal wall and provide a sealed pathway for insertion of surgical instruments into an abdominal cavity. These trocar assemblies typically include an access port or cannula having a housing and an elongate tubular member extending distally from the housing. A channel or lumen extends through the housing and elongate tubular member for receipt of surgical instruments. One or more valves or seals may be provided within the housing to seal against the surgical instruments. The trocar assemblies additionally include a tissue penetrating or incising device or obturator which is positioned through the cannula. The obturator typically includes a tissue penetrating tip at a distal end which, when assembled with the cannula, extends beyond the distal end of the cannula. Advancement of a trocar assembly against an abdominal wall causes the tissue penetrating tip of the obturator to penetrate the abdominal wall and allow passage of the distal end of the cannula into the abdominal cavity.

During insertion of the trocar assembly through the abdominal wall, care must be taken not to damage underlying organs by engagement with the tissue penetrating tip of the obturator or the distal end of the cannula.

Additionally, it often necessary to provide additional access ports through the abdominal wall in order to provide passageways for optical visualization devices such as endoscopes, etc. to remotely view the surgical procedure. Still more access ports may be necessary to provide light to illuminate the operative site and assist in viewing the surgical procedure.

Therefore, there exists a need for a single access port incorporating an optical fiber to visualize the operative site. Further, there exists a need for an access port incorporating both an optical fiber and one or more light fibers to illuminate the operative site.

### SUMMARY

There is disclosed a trocar assembly with integrated optical scope and light fibers. The trocar assembly includes an obturator and an illuminated optical access port for illuminating and visualizing an operative site. The illuminated optical access port generally includes a housing having a distal end, a proximal end and a housing side wall defining a chamber. An elongate tubular member extends distally from the housing and has a proximal end, a distal end and a side wall defining a through bore. An optical fiber extends through the housing sidewall and the sidewall of the elongate tubular member, the optical fiber extending from the distal end of the elongate tubular member to a point within the sidewall of the housing. The optical fiber includes a distal end incorporating an optical lens. A light fiber extends through the housing sidewall and the sidewall of elongate tubular member. The light fiber extends from the distal end of the elongate tubular member to a point within the sidewall of the housing. Alternatively, the light fiber have a sheath and may be located in a channel cut into the housing and elongate tubular member, preferably on the exterior of the housing and member.

In a specific embodiment, an optical lumen is formed through the housing side wall and the side wall of the elongate tubular member and extends from the distal end of the elongate tubular member through the point within the sidewall of the housing. The optical fiber extends through the optical lumen.

In an alternative embodiment, a sheath is positioned between the optical lumen and the optical fiber to enhance transmission of images along the optical fiber.

In a further specific embodiment, a light lumen is formed through the housing side wall and the side wall of the elongate tubular member and extends from the distal end of the elongate tubular member through the point within the sidewall of the housing.

In one embodiment, at least one additional light fiber extends through the housing side wall and the side wall of the elongate tubular member. In a more specific embodiment, at least one additional light lumen is provided extending from a distal end of the elongate tubular member to a proximal end of the sidewall of the housing, wherein the point within the sidewall of the housing is located in the proximal end of the sidewall of the housing.

Alternatively in the various embodiments, the light fiber may not extend to the distal portion of the elongate tubular member. In these embodiments the elongate tubular member may be transparent and serve as a guide to guide the light from the proximal portion of the elongate tubular member to the distal end. The sides and proximal end of the elongate tubular member may be reflective to enhance the transmission of light.

In an alternative embodiment, a light fiber ring is positioned within the proximal end of the sidewall of the housing end in light transmissive contact with a proximal end of the light fiber. The light fiber ring includes a proximal light fiber extending from the light fiber ring to the point within the sidewall of the housing. The sides and proximal end of the light fiber ring may be reflective to enhance the transmission of light.

The illuminated optical access port additionally includes a connector engageable with a port in the sidewall of the housing. The connector incorporates a light driver and an optical driver. The light driver is in a light transmissive contact with the proximal end of the light fiber and the optical driver is in optically transmissive contact with a proximal end of the optical fiber.

There is also disclosed an illuminated optical access port including a housing having a distal end, a proximal end and a housing side wall defining a chamber. An elongate tubular member extends distally from the housing and has a proximal end, a distal end and a side wall defining a through bore. An optical fiber extends through the housing sidewall and the sidewall of the elongate tubular member, the optical fiber extending from the distal end of the elongate tubular member to a point within the sidewall of the housing. The optical fiber includes a distal end incorporating an optical lens. A light fiber extends through the housing sidewall and the sidewall of elongate tubular member. The light fiber extends from the distal end of the elongate tubular member to a point within the sidewall of the housing. A light plate positioned on a proximal end of the housing.

A light fiber ring is positioned within the light plate and is in light transmissive contact with a proximal end of the light fiber. In one embodiment, the light fiber ring includes a proximal light fiber extending from the light fiber ring to the point within the sidewall of the housing.

### DESCRIPTION OF THE DRAWINGS

Various embodiments of the presently disclosed trocar assembly having integrated illuminating light fibers and optical viewing scope fibers are disclosed herein with reference to the drawings, wherein:
FIG. 1 is a perspective view of one embodiment of a trocar assembly having integrated illuminating light fibers and optical viewing scope fibers, with parts separated, including an obturator and an illuminated optical access port;
FIG. 2 is a cross-sectional view of the illuminated optical access port of FIG. 1;
FIG. 3 is a cross-sectional view of an elongate tubular member of the illuminated optical access port taken along line 3-3 of FIG. 2;
FIG. 4 is an enlarged area of detail view of FIG. 3 illustrating an optical fiber shielding sheath;
FIG. 5 is a cross-sectional view of an alternative embodiment of an illuminated optical access port;
FIG. 6 is a cross-sectional view of an alternative embodiment of an elongate tubular member of the illuminated optical access port incorporating a pair of optical scope fibers;
FIG. 7 is a cross-sectional view of the elongate tubular member of the illuminated optical access port of FIG. 5;
FIG. 8 is a cross-sectional view taken along line 8-8 of FIG. 5;
FIG. 9 is an enlarged area of detail view of FIG. 8 illustrating an area of an illuminating fiber ring; and
FIG. 10 is a cross-sectional view taken along line 10-10 of FIG. 5.

### DETAILED DESCRIPTION OF EMBODIMENTS

Embodiments of the presently disclosed trocar assembly with integrated optical and light fibers will now be described in detail with reference to the drawings wherein like numerals designate identical or corresponding elements in each of the several views. As is common in the art, the term 'proximal" refers to that part or component closer to the user or operator, i.e. surgeon or physician, while the term "distal" refers to that part or component further away from the user.

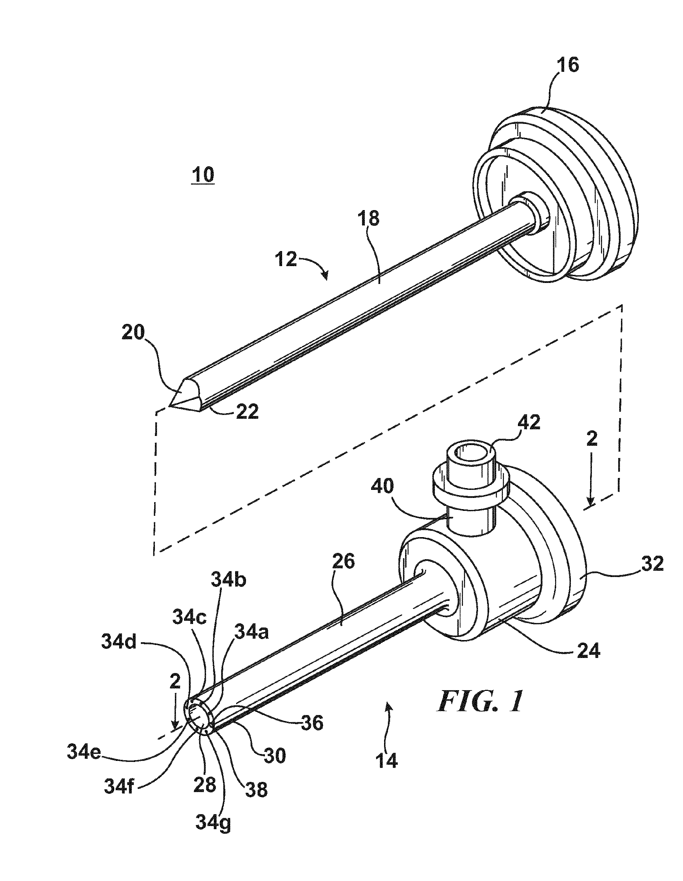

FIG. 1 illustrates one embodiment of the presently disclosed trocar assembly with integrated optical and light fibers or optical trocar 10. Optical trocar 10 generally includes an obturator 12 and an illuminated optical access port or cannula 14 for illumination and visualization of an operative site. Obturator 10 includes a handle 16 having an obturator shaft 18 extending distally from handle 16. A tissue penetrating tip 20 is provided at a distal end 22 of obturator shaft 18.

Cannula 14 includes a housing 24 having an elongate tubular member 26 extending distally of housing 24. A throughbore 28 extends through elongate tubular member 26 and housing 24 and is configured to receive obturator shaft 18 therethrough during initial penetration of tissue. Throughbore 28 extends from a distal end 30 of elongate tubular member 26 to a proximal end 32 of housing 24. A plurality of light fibers 34a-g are arrayed around a distal face 36 of elongate tubular member 26 to illuminate an operative site.

At least one scope or optical fiber 38 also is located in the array in distal face 36 to provide visualization of the operative site. Light fibers 34a-g and optical fiber 38 extend from distal face 36 to a port 40 provided on housing 24. A coupling 42 is associated with drivers for a light source and optical system (not shown) and is engageable with port 40. While not specifically shown, the light source and optical system are of the type commonly known in the art, such as, for example, those used in endoscopes, laparoscopes, etc. Light fibers 34a-g and optical fiber 38 are integrated into elongate tubular member 26 and housing 24 in a manner described in more detail herein below. Additionally, while not specifically shown, housing 24 may be provided with an insufflation valve common to cannula access port systems to receive a source of insufflation fluid to insufflate the operative site.

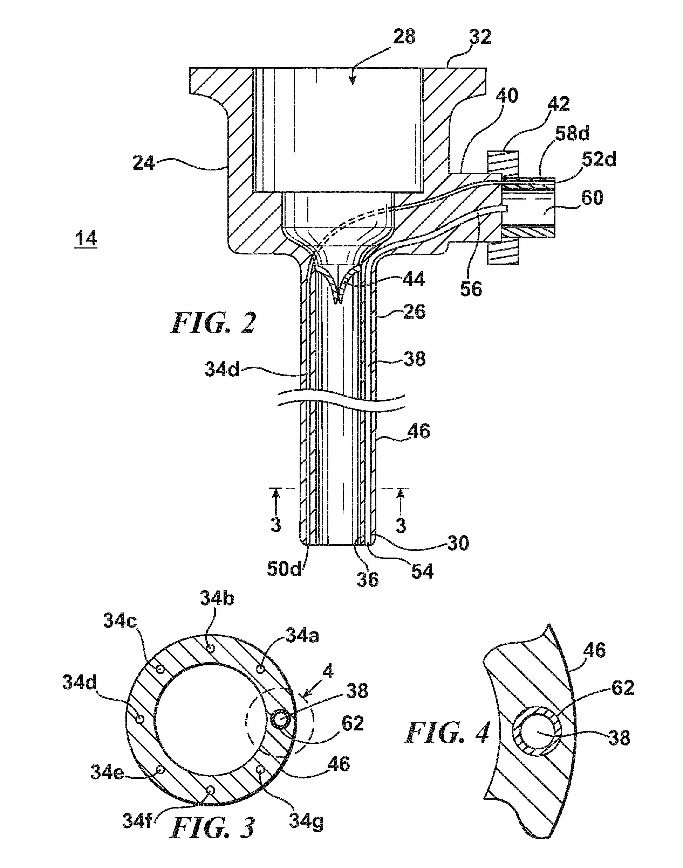

Referring now to FIGS. 2-4, and initially with regard to FIG. 2, as noted above, throughbore 28 extends through cannula 14 from distal end 30 of elongate tubular member 26 to proximal end 32 of housing 24. One or more seals or valves 44 may be provided in elongate tubular member 26 or housing 24 to seal about a surgical instrument inserted therethrough in order to prevent loss of insufflation fluid. Light fibers 34a-g and optical fiber 38 extend through a side wall 44 of elongate tubular member 26 and a side wall 46 of housing 24. Distal ends 50a-g of light fibers 34a-g are open to distal face 36 of elongate tubular member 26 while proximal ends 52a-g of light fibers 34a-g terminate in port 40. Similarly, a distal end 54 of optical fiber 38 is open to distal face 36 of elongate tubular member while a proximal end 56 of optical fiber 38 terminates in port 40. While not specifically shown, distal end 54 of optical fiber 38 may be provided with any of several optical lenses, such as, for example, fish eye, wide or narrow view, angled view, etc. to enhance visualization of the operative site. Proximal ends 52a-g of light fibers 34a-g communicate with light fibers 58a-d in connector 42 and proximal end 56 of optical fiber 38 communicates with a optical fiber 60 in connector 42.

Light fibers 34a-g and optical fiber 38 are built integrally into side walls 46 and 48 of elongate tubular member 26 and housing 24, respectively. This may be accomplished by molding housing 24 and elongate tubular member directly around light fibers 34a-g and optical fiber 38 to directly integrate them together. Alternatively, optical lumens (not shown) may be formed through side walls 46 and 48 of elongate tubular member 26 and housing 24 to receive optical fiber 38. Similarly, light lumens (not shown) may be formed through side walls 46 and 48 for receipt of light fibers 34a-g.

Referring for the moment to FIGS. 3 and 4, a sheath 62 may be incorporated between optical fiber 38 and side wall 46 of elongate tubular member 26 and side wall 48 of housing 24 (not shown). Sheath 62 is provided to prevent distortion of images by side walls 46 and 48 as the images pass through optical fiber 38.

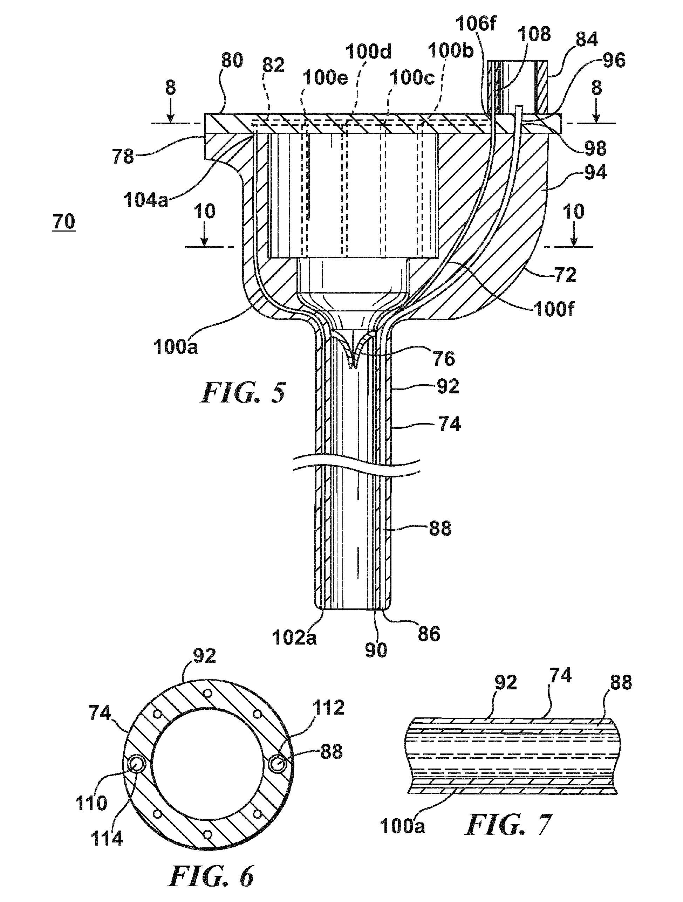

Referring now to FIGS. 5 and 7-10, an alternative embodiment of an illuminated optical access port or cannula 70 will now be described. Cannula 70 includes a housing 72 and an elongate tubular member 74 extending distally from housing 72. Similar to cannula 14 described herein above, cannula 70 may be provided with one or more seals or valves 76 to seal about surgical instruments and an insufflation port to provide a source of insufflations fluid. Unlike cannula 14 above, a proximal end 78 of housing 72 is provided with a light plate 80 incorporating a light ring 82 as discussed in more detail herein below. A port 84 is provided on light ring for connection with a light source and optical connection similar to that described hereinabove with regard to coupling 42.

A distal end 86 of an optical fiber 88 extends from a distal face 90 of elongate tubular member 74, through a side wall 92 of elongate tubular member 74 and through a side wall 94 of housing 72. A passageway 96 in light plate 80 is provided to receive a proximal end 98 of optical fiber 88 which is in communication with port 84.

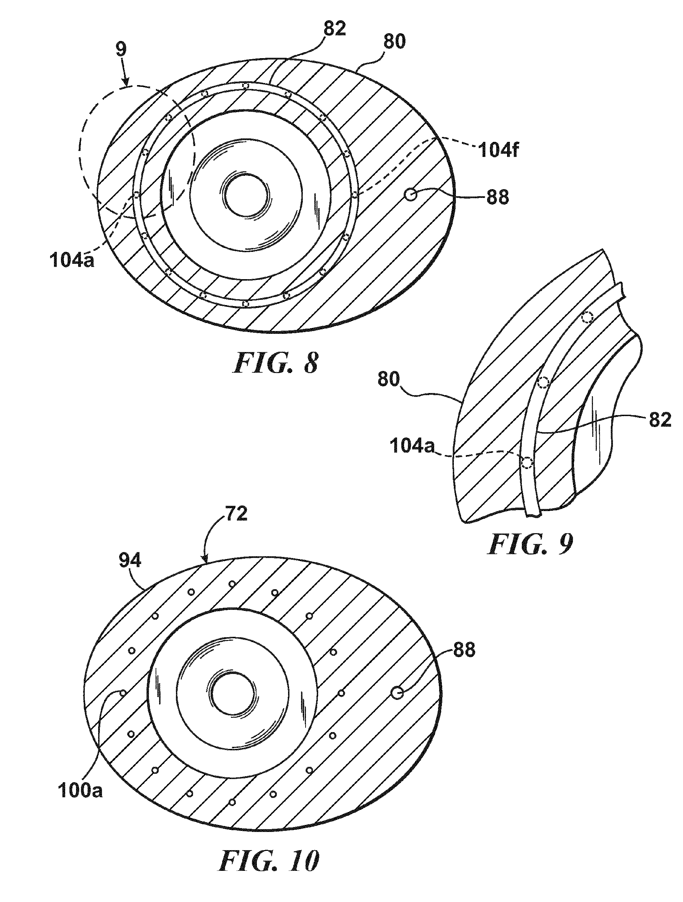

Cannula 70 is also provided with one or more light fibers 100a-x, depending on the number of light fibers desired. While the following description is given with regard to light fiber 100a, all the disclosed light fibers 100s function identically. Light fiber 100a extends through side walls 92 and 94 of elongate tubular member74 and 72, respectively (see also, FIG. 10). A distal end 102a of light fiber 100a is open to distal face 90 of elongate tubular member 74.

Referring now to FIGS. 5, 8 and 9, a proximal end 104f of light fiber 102a passes through light plate 80 and is connected to, and in communication with, light ring 82 in light plate 80. Referring for the moment to light fiber 100f, a proximal end 106f of light fiber 100f passes through light plate 80 and extends into port 84 to provide a source of light to light ring 82 and thus to all the disclosed light fibers 100a-x, etc. It should be noted that, while light ring 82 is described as being integrated into light plate 80, light plate 80 may alternatively be provided with a light lumen (not shown) for receipt of light ring 82. Alternatively, proximal end 106f of light ring 100f may terminate at light ring 82 and a separate light fiber or tag 108 may extend from light ring 82 and into port 84 to provide a source of light to light ring 82.

Additionally, while proximal end 106f of light fiber 100f is shown in radially spaced relation to proximal end 98 of optical fiber 88, they may be positioned in side by side relation to maintain a constant wall thickness of side wall 94 of housing 72.

Referring back for the moment to FIG. 6, in an alternative embodiment, cannula 70 may be provided with a second optical fiber 110. Optical fibers 88 and 110 may be directly incorporated into side wall 92 of elongate tubular member 74 or may be insulated therefrom by sheaths 112 and 114 surrounding optical fibers 88 and 110, respectively.

It will be understood that various modifications may be made to the embodiments disclosed herein. For example, sheaths may be provided about the light fibers to prevent scatter. Additionally, the disclosed light ring may be incorporated directly into the proximal end of the cannula housing. Therefore, the above description should not be construed as limiting, but merely as exemplifications of particular embodiments. Those skilled in the art will envision other modifications within the scope and spirit of the claims appended hereto. The invention may be described be reference to the following numbered paragraphs:-
1. An illuminated optical access port comprising:
   a housing having a distal end and a proximal end, the housing having a housing side wall defining a chamber;
   an elongate tubular member extending distally from the housing and having a proximal end and a distal end, the elongate tubular member having a side wall defining a through bore;
   an optical fiber extending through the housing sidewall and the sidewall of the elongate tubular member, the optical fiber extending from the distal end of the elongate tubular member to a point within the sidewall of the housing; and
   a light fiber extending through the housing sidewall and the sidewall of elongate tubular member, the light fiber extending from the distal end of the elongate tubular member to a point within the sidewall of the housing.
2. The illuminated optical access port as recited in paragraph 1, further comprising an optical lumen formed through the housing side wall and the side wall of the elongate tubular member and extending from the distal end of the elongate tubular member through the point within the sidewall of the housing, the optical fiber extending through the optical lumen.
3. The illuminated optical access port as recited in paragraph 1, wherein the optical fiber includes a distal end incorporating an optical lens.
4. The illuminated optical access port as recited in paragraph 2, further comprising a sheath positioned between the optical lumen and the optical fiber.
5. The illuminated optical access port as recited in paragraph 1, further comprising a light lumen formed through the housing side wall and the side wall of the elongate tubular member and extending from the distal end of the elongate tubular member through the point within the sidewall of the housing.
6. The illuminated optical access port as recited in paragraph 1, further comprising at least one additional light fiber extending through the housing side wall and the side wall of the elongate tubular member.
7. The illuminated optical access port as recited in paragraph 6, further comprising at least one additional light lumen extending from a distal end of the elongate tubular member to a proximal end of the sidewall of the housing, wherein the point within the sidewall of the housing is located in the proximal end of the sidewall of the housing.
8. The illuminated optical access port as recited in paragraph 6, further comprising a light fiber ring positioned within the proximal end of the sidewall of the housing end in light transmissive contact with a proximal end of the light fiber and a proximal end of the at least one additional light fiber, the light fiber ring including a proximal light fiber extending from the light fiber ring to the point within the sidewall of the housing.
9. The illuminated optical access port as recited in paragraph 8, further comprising a connector engageable with the sidewall of the housing, the connector incorporating a light driver and an optical driver, wherein the light driver is in a light transmissive contact with the proximal light fiber and the optical driver is in optically transmissive contact with a proximal end of the optical fiber.
10. The illuminated optical access port as recited in paragraph 5, further comprising a connector engageable with the sidewall of the housing, the connector incorporating a light driver and an optical driver, wherein the light driver is in light transmissive contact with a proximal end of the light fiber and the optical driver is in optically transmissive contact with a proximal end of the optical fiber.
11. An illuminated optical access port comprising:
   a housing having a distal end and a proximal end, the housing having a housing side wall defining a chamber;
   an elongate tubular member extending distally from the housing and having a proximal end and a distal end, the elongate tubular member having a side wall defining a through bore;
   an optical fiber extending through the housing sidewall and the sidewall of the elongate tubular member, the optical fiber extending from the distal end of the elongate tubular member to a point within the sidewall of the housing;
   a light fiber extending through the housing sidewall and the sidewall of elongate tubular member, the light fiber extending from the distal end of the elongate tubular member to a point within the sidewall of the housing; and
   a light plate positioned on a proximal end of the housing.
12. The illuminated optical access port as recited in paragraph 11, further comprising a light fiber ring positioned within the light plate and in light transmissive contact with a proximal end of the light fiber, the light fiber ring including a proximal light fiber extending from the light fiber ring to the point within the sidewall of the housing.

## Claims

1. An illuminated optical access port comprising:
a housing having a distal end and a proximal end, the housing having a housing side wall defining a chamber;
an elongate tubular member extending distally from the housing and having a proximal end and a distal end, the elongate tubular member having a side wall defining a through bore;
an optical fiber extending through the housing sidewall and the sidewall of the elongate tubular member, the optical fiber extending from the distal end of the elongate tubular member to a point within the sidewall of the housing; and
a light fiber extending through the housing sidewall and the sidewall of elongate tubular member, the light fiber extending from the distal end of the elongate tubular member to a point within the sidewall of the housing.

2. The illuminated optical access port as recited in claim 1, further comprising an optical lumen formed through the housing side wall and the side wall of the elongate tubular member and extending from the distal end of the elongate tubular member through the point within the sidewall of the housing, the optical fiber extending through the optical lumen.

3. The illuminated optical access port as recited in claim 1 or claim 2, wherein the optical fiber includes a distal end incorporating an optical lens.

4. The illuminated optical access port as recited in any preceding claim, further comprising a sheath positioned between the optical lumen and the optical fiber.

5. The illuminated optical access port as recited in any preceding claim, further comprising a light lumen formed through the housing side wall and the side wall of the elongate tubular member and extending from the distal end of the elongate tubular member through the point within the sidewall of the housing.

6. The illuminated optical access port as recited in any preceding claim, further comprising at least one additional light fiber extending through the housing side wall and the side wall of the elongate tubular member.

7. The illuminated optical access port as recited in claim 6, further comprising at least one additional light lumen extending from a distal end of the elongate tubular member to a proximal end of the sidewall of the housing, wherein the point within the sidewall of the housing is located in the proximal end of the sidewall of the housing.

8. The illuminated optical access port as recited in any preceding claim, further comprising a light fiber ring positioned within the proximal end of the sidewall of the housing end in light transmissive contact with a proximal end of the light fiber and a proximal end of the at least one additional light fiber, the light fiber ring including a proximal light fiber extending from the light fiber ring to the point within the sidewall of the housing.

9. The illuminated optical access port as recited in claim 8, further comprising a connector engageable with the sidewall of the housing, the connector incorporating a light driver and an optical driver, wherein the light driver is in a light transmissive contact with the proximal light fiber and the optical driver is in optically transmissive contact with a proximal end of the optical fiber.

10. The illuminated optical access port as recited in claim 5, further comprising a connector engageable with the sidewall of the housing, the connector incorporating a light driver and an optical driver, wherein the light driver is in light transmissive contact with a proximal end of the light fiber and the optical driver is in optically transmissive contact with a proximal end of the optical fiber.

11. An illuminated optical access port comprising:
a housing having a distal end and a proximal end, the housing having a housing side wall defining a chamber;
an elongate tubular member extending distally from the housing and having a proximal end and a distal end, the elongate tubular member having a side wall defining a through bore;
an optical fiber extending through the housing sidewall and the sidewall of the elongate tubular member, the optical fiber extending from the distal end of the elongate tubular member to a point within the sidewall of the housing;
a light fiber extending through the housing sidewall and the sidewall of elongate tubular member, the light fiber extending from the distal end of the elongate tubular member to a point within the sidewall of the housing; and
a light plate positioned on a proximal end of the housing.

12. The illuminated optical access port as recited in claim 11, further comprising a light fiber ring positioned within the light plate and in light transmissive contact with a proximal end of the light fiber, the light fiber ring including a proximal light fiber extending from the light fiber ring to the point within the sidewall of the housing.
